# FASCICULE DE BREVET EUROPEEN

(11) **EP 0 759 022 B1**
(45) Date de publication et mention de la délivrance du brevet: **26.01.2000**
(21) Numéro de dépôt: 95920140.1
(22) Date de dépôt: 15.05.1995
(51) Int. Cl.: C07C 51/12, C07C 67/36, C07C 51/47, C07C 67/56

(54) **PROCEDE DE PREPARATION D'ACIDES CARBOXYLIQUES OU DES ESTERS CORRESPONDANTS EN PRESENCE D'UN CATALYSEUR A BASE D'IRIDIUM ET D'IODURES SOUS FORME SOLUBLE**
VERFAHREN ZUR HERSTELLUNG VON CARBONSÄUREN ODER IHRER ESTER IN GEGENWART EINES AUF IRIDIUM BASIERTEN LÖSLICHEN KATALYSATORS UND LÖSLICHER JODIDE
METHOD FOR THE PREPARATION OF CARBOXYLIC ACIDS OR CORRESPONDING ESTERS IN THE PRESENCE OF AN IRIDIUM-BASED CATALYST AND SOLUBLE IODIDES

(30) Priorité: 13.05.1994 FR 9405896; 21.10.1994 FR 9412712
(43) Date de publication de la demande: 26.02.1997
(73) Titulaire: ACETEX CHIMIE, 92082 Paris La Défense 2 (FR)
(72) Inventeur: DENIS, Philippe, F-69150 Decines (FR); NOBEL, Dominique, F-30140 Salindres (FR); PERRON, Robert, F-69390 Charly (FR); PERRONA, Philippe, F-69003 Lyon (FR); SCHWARTZ, Joel, F-69300 Caluire (FR)
(74) Mandataire: Giraud, Françoise
(86) Numéro de dépôt international: FR9500625
(87) Numéro de publication internationale: WO9531426

(56) Documents cités:
- EP-A- 0 196 173
- EP-A- 0 484 020
- EP-A- 0 618 184
- US-A- 3 772 380

## Description

La présente invention concerne un procédé de préparation d'acides carboxyliques ou des esters correspondants, par carbonylation, en phase liquide, d'un alcool, en présence d'un catalyseur à base d'iridium.

L'obtention d'acides carboxyliques, et plus particulièrement d'acide acétique, par réaction de monoxyde de carbone avec un alcool comme le méthanol, en présence d'un catalyseur homogène est un procédé bien connu, ayant fait l'objet de nombreux brevets et articles. Les catalyseurs susceptibles d'être utilisés dans ce type de réaction sont entre autres le cobalt, le rhodium et l'iridium.

Seuls les procédés de carbonylation utilisant des catalyseurs à base de rhodium font actuellement l'objet de nouveaux développements à une échelle industrielle. Les dernières générations de ces procédés mettent ainsi en oeuvre le rhodium, des quantités importantes de sels solubles d'iodures stabilisant le métal précité et de faibles teneurs en eau. Ils sont très performants puisqu'ils permettent d'atteindre des vitesses de carbonylation du méthanol en acide acétique supérieures à 10 mol/l.h.

La préparation d'acides carboxyliques catalysée par le cobalt n'est plus retenue actuellement pour faire l'objet de nouvelles exploitations du fait des conditions de réaction très dures en pression et en température. Malgré de telles conditions les résultats obtenus, en terme de sélectivité en acide formé, sont relativement peu satisfaisants, en comparaison des contraintes à gérer.

Quant aux procédés utilisant une catalyse à base d'iridium, les résultats mis en évidence sont très peu performants. En effet, les vitesses de carbonylation rapportées sont de l'ordre de 2 à 4 mol/h.l d'acide formé alors que le nombre de moles d'iridium engagé dans la réaction est très important. De tels procédés ne sont pas exploitables de façon satisfaisante sur le plan industriel.

Par exemple le brevet U.S. 3 772 380 décrit la préparation d'acides carboxyliques et de leurs esters par réaction d'alcools et de leur dérivés de type esters, éthers ou dérivés halogénés, avec le monoxyde de carbone en présence de systèmes catalytiques contenant comme constituants actifs un composé de l'iridium et un composé halogéné.

Dans la demande de brevet européen EP 618 184, est décrit un procédé de carbonylation d'un alcool, tel que le méthanol, en présence d'un système catalytique à base d'iridium et d'un promoteur halogéné, amélioré par rapport au procédé décrit ci-dessus. Il a été trouvé qu'en maintenant une composition particulière du mélange réactionnel, comprenant jusqu'à 10 % en eau, en alcool, en promoteur halogéné, et jusqu'à 40 % en ester correspondant à l'alcool et à l'acide, il était possible d'atteindre des vitesses de carbonylation pouvant supérieures ou égales à 10 mol/h.l d'acide formé. De telles vitesses de carbonylation sont tout à fait comparables à celles obtenues en mettant en oeuvre le procédé de carbonylation catalysé au rhodium.

La demande de brevet européen EP 0 657 386 décrit un procédé de préparation d'une solution à base d'iridium utilisable en particulier en tant que catalyseur de carbonylation, selon lequel on met en contact , en phase liquide, au moins un oxyde ou un hydroxyde, hydraté ou non, d'iridium, ou leur mélange, en présence d'un solvant, avec de l'acide iodhydrique ou un composé susceptible de libérer de l'acide iodhydrique, dans une quantité telle que le nombre de moles d'acide iodhydrique rapporté au nombre de moles d'iridium soit compris entre 1 et 100.

La présente invention a donc pour but de proposer un mode de préparation d'acides carboxyliques par une réaction de carbonylation d'un réactif approprié, ne mettant pas en oeuvre un catalyseur à base de rhodium, tout en conservant une productivité comparable à celles des procédés utilisant le catalyseur précité.

Ainsi, le procédé selon l'invention a pour objet l'obtention d'acides carboxyliques ou des esters correspondants, présentant (n+1) atomes de carbone, par réaction en phase liquide, de monoxyde de carbone avec au moins un alcool présentant n atomes de carbone, en présence d'un système catalytique à base d'un composé de l'iridium et d'un promoteur halogéné. Le procédé selon l'invention, en outre, est caractérisé par le maintien, dans le milieu, pendant la réaction, de l'eau dans une teneur variant entre 0 exclu et 10 %, du promoteur halogéné dans une teneur variant entre exclu 0 et 10%, de l'ester correspondant à l'acide carboxylique et l'alcool précité, dans une teneur variant entre 2 et 40 %, d'iodures sous forme solubles, de telle sorte que le rapport atomique des iodures à l'iridium soit compris entre 0 exclu et 10, l'acide carboxylique précité constituant le solvant de la réaction.

Dans tout ce qui va suivre, et sauf indication contraire, les pourcentages indiqués sont exprimés en poids, rapportés au poids total du mélange réactionnel.

Il a été trouvé de façon totalement surprenante que le procédé selon l'invention, réalisé en présence d'un catalyseur à base d'iridium dans les conditions stables explicitées ci-dessus, est beaucoup plus performant que les procédés décrits mettant en oeuvre un tel catalyseur. De plus, et ce fait est important, le procédé selon l'invention permet d'atteindre des vitesses de carbonylation de l'alcool en acide carboxylique, exprimées en moles, comparables à celles obtenues avec les procédés catalysés par le rhodium, en mettant en oeuvre des quantités de catalyseur similaires.

Il est de même à noter que le procédé selon l'invention est mis en oeuvre en présence de quantités relativement faibles de promoteur halogéné. Ceci présente les avantages de diminuer la quantité de promoteur à séparer de l'acide formé et de diminuer la consommation d'énergie nécessaire à la récupération de ce composé halogéné ainsi que sa consommation spécifique, c'est-à-dire sa consommation lors d'une exploitation en continu du procédé de carbonylation.

Par ailleurs, cette mesure a permis de baisser, pour des teneurs en eau et en ester données, la teneur en hydracide correspondant à l'halogène du promoteur dans le milieu. Par conséquent la corrosivité dudit milieu est diminuée, rendant plus facile et moins coûteux le choix des matériaux, mis en contact avec un tel milieu.

On a enfin constaté que l'iridium mis en oeuvre dans de telles conditions est remarquablement sélectif, en ce sens que la quantité de sous-produits formés, comme par exemple l'acide propionique, l'acide formique, est très faible.

En effet, la quantité des deux acides précités formés pendant la réaction est, en général, pour chacun d'eux, inférieure à 200 ppm.

Mais d'autres buts et avantages de la présente invention apparaîtront plus clairement à la lecture de la description qui va suivre.

Ainsi qu'il a été dit précédemment, la réaction de carbonylation de l'invention est réalisée en présence d'un système catalytique à base d'au moins un composé de l'iridium et d'un promoteur halogéné.

La réaction étant effectuée en phase liquide, le système catalytique se présente sous forme de composés solubles dans le mélange réactionnel.

Tous les composés de l'iridium, solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention, l'iridium à l'état métallique, les sels simples de ce métal, les oxydes ou encore les complexes de coordination. Pour plus de détail, on pourra notamment se référer au brevet américain US 3 772 380 dans laquelle figure une liste de tels composés.

De préférence, on utilise des sels simples d'iridium, comme les halogénures d'iridium, l'halogène étant plus particulièrement choisi parmi le chlore, le brome ou de préférence l'iode.

Les oxydes d'iridium de même que les complexes solubles de coordination de l'iridium, conviennent à la mise en oeuvre de l'invention. Dans cette dernière catégorie les composés les plus couramment employés sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles d'iridium dont les ligands sont choisis parmi des composés organo-phosphorés ou organo-azotés, par exemple.

Ces catalyseurs peuvent être obtenus par toute méthode connue de l'homme du métier.

Cependant, selon un mode particulièrement avantageux, on peut préparer une solution catalytique à partir d'un complexe carbonylé d'iridium, tel que Ir₄CO₁₂, par mise en contact dudit composé avec de l'acide iodhydrique et/ou un précurseur d'un tel acide, en présence d'un solvant.

Comme précurseur susceptible de libérer l'acide iodhydrique, on peut mentionner, à titre d'exemple, l'iode, les iodures d'alkyle en C₁-C₁₀, ou encore les iodures d'alcoyles en C₁-C₁₀, les iodures de métaux alcalins.

En ce qui concerne les solvants, tous les composés peuvent être utilisés dans la mesure où ils solubilisent l'acide iodhydrique ou son précurseur et le composé à base d'iridium obtenu. Plus particulièrement on met en oeuvre des solvants, seuls ou en mélange, choisis parmi les acides carboxyliques ou les esters correspondants, obtenus par le procédé selon l'invention, ou l'eau.

La mise en contact a lieu sous une pression totale comprise entre 1 et 10 bar, à une température au plus égale à la température d'ébullition du solvant précité, dans les conditions de la mise en contact.

L'opération peut être effectuée sous air, sous un gaz inerte ou encore sous monoxyde de carbone.

Un autre exemple de méthode avantageuse pour la préparation d'une solution catalytique convenable à la mise en oeuvre de l'invention, consiste à mettre en contact, en phase liquide, un ou plusieurs oxydes hydratés ou non d'iridium, avec de l'acide iodhydrique ou un composé susceptible de libérer de l'acide iodhydrique. L'acide iodhydrique peut être employé sous la forme d'un gaz, d'une solution, plus particulièrement aqueuse. Il peut encore être utilisé sous la forme d'un précurseur, comme notamment ceux mentionnés dans la précédente variante.

Plus particulièrement la quantité d'acide iodhydrique est telle que le rapport entre le nombre de moles d'acide iodhydrique rapporté au nombre de moles d'iridium, varie entre 1 et 100.

Le procédé selon l'invention peut être mis en oeuvre sous air, sous un gaz inerte, sous monoxyde de carbone, ces gaz étant seuls ou en combinaison.

Généralement, la concentration totale en iridium dans le milieu réactionnel engagé, dans le procédé selon l'invention, est comprise entre 0,1 et 100 mmol/l.

Selon un mode de réalisation particulier, la concentration en iridium varie de 0,5 à 40 mmol/l et de préférence entre 1 et 25 mmol/l.

Le second constituant du système catalytique est un promoteur halogéné. Celui-ci peut se présenter sous la forme d'un halogène seul, ou en combinaison avec d'autres éléments comme par exemple l'hydrogène, un radical alkyle en C₁-C₁₀, un radical acyle en C₁-C₁₀, un radical aryle en C₆-C₁₀.

L'halogène est en général choisi parmi le chlore, le brome ou l'iode, ce dernier étant préféré.

Selon un mode de réalisation particulier de l'invention, le promoteur mis en oeuvre comprend l'hydrogène ou un radical alkyle en C₁-C₁₀. Plus particulièrement, le promoteur utilisé dans l'invention comprend l'halogène et un radical alkyle en C₁-C₁₀.

De préférence, ce mode de réalisation est effectué en présence d'un promoteur halogéné dont le radical correspond à celui de l'alcool utilisé comme réactif lors de la réaction selon l'invention.

La teneur en promoteur halogéné dans le milieu est comprise entre 0 exclu et 10 %. Selon une variante de l'invention, la teneur en composé halogéné dans le milieu réactionnel est comprise entre 0,5 et 8 % et de préférence entre 1 et 6 %.

Comme cela a été mentionné auparavant, la réaction selon l'invention est effectuée en présence d'un alcool comprenant un atome de carbone en moins par rapport à l'acide carboxylique, ou à l'ester correspondant, fabriqué.

Parmi les réactifs convenables à la mise en oeuvre de la réaction, on peut citer les alcools saturés, présentant un à dix atomes de carbone. A titre d'exemple de tels composés, on peut citer notamment le méthanol, l'éthanol, le propanol, le butanol, le 1,4-butanediol. Ces alcools peuvent être mono- ou di- hydroxylés.

Selon un mode de réalisation préféré, les alcools utilisés sont choisis parmi les composés monohydroxylés.

Il est important de noter que l'alcool utilisé comme réactif peut être présent dans le milieu réactionnel en tant que tel ou sous forme masquée. En effet, ledit alcool peut se trouver indifféremment sous la forme d'un dérivé halogéné et/ou d'un éther et/ou d'un ester obtenu par réaction entre ledit alcool et l'acide carboxylique présent.

Ainsi, la teneur en réactif dans le milieu réactionnel peut varier dans de larges limites, du fait des différentes espèces sous lesquelles le réactif peut se présenter.

Par conséquent, la teneur en alcool en tant que tel, dans le milieu réactionnel peut être comprise entre 0 et 10 %.

De préférence, le milieu comprend une teneur en alcool comprise entre 0,1 et 8 %.

L'autre réactif nécessaire à l'obtention d'un acide carboxylique est le monoxyde de carbone. Celui-ci peut être utilisé sous forme pure ou diluée dans des gaz tels que l'hydrogène, le méthane, le dioxyde de carbone, ou tout autre type de gaz comme par exemple l'azote.

Selon un mode particulier de réalisation de l'invention, on utilise un monoxyde de carbone présentant une pureté d'au moins 99 %.

La pression partielle en monoxyde de carbone est habituellement comprise entre 5 et 200 bar, plus particulièrement entre 5 et 100 bar. De préférence, la pression partielle en monoxyde de carbone est comprise entre 10 et 50 bar. Il faut cependant noter que des pressions partielles en dehors de ces gammes restent envisageables.

La réaction de carbonylation selon l'invention est effectuée en outre, en présence d'eau. La teneur en eau dans le milieu réactionnel est plus particulièrement comprise entre 0 exclu et 10 %.

Selon un mode de réalisation particulier de l'invention, la teneur en eau dans le milieu est comprise entre 0,5 et 8 % et de préférence entre 2 et 8 %.

Outre les composés et réactifs précédemment mentionnés, le procédé selon l'invention est réalisé en présence d'esters correspondant, de préférence, à la réaction de l'alcool mis en jeu dans la réaction, avec l'acide carboxylique présent dans le milieu réactionnel.

Plus particulièrement, la teneur en ester dans ledit milieu est compnse entre 2 et 40%.

Selon un mode de réalisation particulier de l'invention, la teneur en ester est comprise entre 5 et 30%.

Le procédé selon l'invention est effectué dans un solvant qui, de préférence, correspond à l'acide carboxylique formé par la réaction.

Le procédé selon l'invention est enfin mis en oeuvre en présence d'iodures sous forme soluble dans le milieu réactionnel. Les iodures peuvent être introduits en tant que tels dans le milieu réactionnel mais aussi sous la forme de composés susceptibles de former des iodures solubles.

Par iodures, on entend des espèces ioniques, c'est-à-dire ne comprenant pas les iodures covalents (tels que notament le promoteur halogéné) ni l'acide iodhydrique.

Ainsi, les iodures introduits dans ledit mélange, en tant que tels, sont choisis parmi les iodures minéraux ou organiques

A titre d'iodures minéraux, on peut citer principalement les iodures de métal alcalino-terreux ou alcalin, ces derniers étant préférés. On peut citer parmi ceux-ci l'iodure de potassium, l'iodure de lithium, l'iodure de sodium.

A titre d'iodures organiques, on peut citer les composés organiques, comprenant au moins un groupe organo-phosphoré et/ou au moins un groupe organo-azoté, réagissant avec des composés à base d'iode, pour donner des espèces ioniques renfermant cet halogène. A titre d'exemple, on peut mentionner l'iodure de triphényle phosphonium, l'iodure de N-méthyltriéthyle ammonium.

A titre de composés susceptibles de former des iodures solubles dans le milieu réactionnel, on peut citer par exemple les carboxylates, les hydroxydes de métaux alcalins ou alcalino-terreux, tels que l'acétate de lithium, la potasse, la soude notamment.

En outre, il est à noter que les iodures peuvent avoir d'autres origines que celles indiquées ci-dessus.

Ainsi, ces composés peuvent provenir d'impuretés comme les métaux alcalins ou alcalino-terreux, impuretés présentes dans les matières premières employées pour préparer la solution catalytique.

Les iodures peuvent de même provenir des métaux de corrosion apparaissant pendant la réaction de carbonylation. Il est préférable de maintenir le seuil de concentration de ces métaux relativement faible, de l'ordre de quelques centaines de parties par millions, car ils ont notamment pour effet de favoriser la réaction de gaz à l'eau et contribuent à augmenter le rapport atomique iodures / iridium.

Une caractéristique importante du procédé de l'invention consiste à introduire dans le milieu réactionnel une quantité d'iodure particulière selon la quantité d'iridium présent dans le milieu. Ainsi, cette quantité d'iodures introduite est telle que le rapport atomique iodures introduits/iridium (exprimé en mole/mole), est compris entre 0 exclu et 10 et à maintenir ce rapport dans la gamme indiquée, pendant la réaction.

Selon un mode de réalisation préféré de l'invention, le rapport atomique iodures/iridium est maintenu entre 0 exclu et 3. Plus particulièrement, ce rapport est compris entre 0 exclu et 1,5.

On a trouvé que l'ajout de telles quantités d'iodures permet d'améliorer la stabilité du catalyseur et de conserver une productivité importante au procédé.

Ainsi que cela a été indiqué auparavant, la présente invention consiste à maintenir dans le milieu réactionnel, l'eau, le promoteur halogéné, l'ester précité, les iodures et l'acide carboxylique, dans les proportions venant d'être explicitées.

Par conséquent, la présente invention est plus particulièrement destinée à être mise en oeuvre en continu et les conditions stables de fonctionnement du procédé correspondent à la composition et aux proportions indiquées.

Plus particulièrement, en ce qui concerne les iodures solubles, le maintien du rapport atomique iodures solubles / iridium peut être effectué en traitant un mélange comprenant au moins le composé de l'iridium, avec une résine échangeuse d'ions puis en ajoutant des iodures sous forme soluble dans une quantité telle que ledit rapport atomique dans le mélange réactionnel soit compris entre 0 exclu et 10.

Une première variante de l'invention consiste à traiter un mélange correspondant à la solution catalytique. Par solution catalytique, on entend la solution comprenant un composé de l'iridium en présence du ou des solvants ou réactifs appropriés.

Selon une seconde variante, le mélange à traiter correspond au mélange réactionnel auquel on n'a pas encore ajouté d'iodures sous forme soluble.

Selon une troisième variante, le mélange à traiter correspond au mélange réactionnel pendant la réaction de carbonylation ou après qu'elle ait été arrêtée. Ainsi, plus particulièrement, le mélange à traiter peut être au moins une partie du flux liquide provenant de la vaporisation partielle du mélange réactionnel.

Il est rappelé à ce sujet que le procédé de carbonylation selon l'invention peut être convenablement mis en oeuvre dans les installations exploitant les procédés classiques. Ces dernières sont habituellement constituées de trois zones. La première correspond à la zone de réaction, comprenant un réacteur sous pression ; la seconde est celle de séparation de l'acide, ou de l'ester, formé, par vaporisation partielle du mélange réactionnel. La partie vaporisée est ensuite envoyée dans une troisième zone, celle de purification de l'acide carboxylique ou de l'ester correspondant ; la partie du mélange restée sous forme liquide, comprenant principalement le catalyseur, est recyclée au réacteur.

La combinaison des variantes précédentes est bien entendu envisageable.

Le mélange à traiter peut l'être en une étape ou non. Ainsi, on peut traiter la totalité du mélange sur la résine. Ceci peut notamment être mis en oeuvre dans le cas où le mélange est par exemple la solution catalytique. Une opération de ce type est en général réalisée en discontinu. On peut aussi envisager écarter une partie du mélange, et traiter ce flux selon l'invention, en continu ou en discontinu. Ceci est de préférence mis en oeuvre lorsque l'on mène la réaction de carbonylation simultanément avec le traitement sur la résine.

Les résines convenant à la mise en oeuvre de l'invention sont plus particulièrement des résines échangeuses de cations, de type acide fort ou acide faible, sous la forme hydrogène.

A titre d'exemple de résines du type acide faible, on peut citer les résines qui sont des copolymères d'acide acrylique, méthacrylique, des esters ou encore des nitriles correspondants. On peut de même citer les résines phénoliques.

Parmi les résines de type acide fort, on peut mentionner tout particulièrement les résines étant des copolymères de styrène divinylbenzène présentant des groupements fonctionnels greffés sulfonés. De telles résines sont commercialisées notamment sous la dénomination commerciale DOWEX de la société Dow, ou encore PUROLITE de la société Purolite, AMBERLYST de la société Rohm & Haas.

Selon un mode de réalisation préféré, on utilise des résines du type fortement acide.

Les résines sont employées indifféremment sous la forme d'un gel ou sous une forme macroporeuse.

Par ailleurs, le traitement du mélange est réalisé plus particulièrement en lit fixe.

La température à laquelle est effectuée la mise en contact du mélange à traiter avec la résine est comprise entre 10 et 150 °C et plus particulièrement entre 20 et 100°C.

Une fois le mélange traité, on ajoute des ions iodures ou leur précurseur, de telle sorte à obtenir un rapport atomique iodure / iridium dans le mélange réactionnel compris dans les gammes données auparavant.

Cette addition peut être effectuée au mélange venant d'être traité mais aussi au mélange réactionnel en général, c'est-à-dire en tout point du procédé où ledit mélange réactionnel existe.

Ainsi, on peut ajouter les iodures ou leur précurseur en tout point de la zone de réaction ou encore de la zone de séparation.

Lors du démarrage de la réaction, les divers composants sont introduits dans un réacteur approprié, muni de moyens d'agitation suffisants pour assurer le transfert gaz-liquide. Il est à noter que si le réacteur comprend de préférence des moyens d'agitation mécanique du mélange réactionnel, il n'est pas exclu d'opérer sans de tels moyens ; l'homogénéisation du mélange pouvant être réalisée par l'introduction du monoxyde de carbone dans le réacteur.

Les composants du milieu réactionnel sont introduits sans ordre préférentiel, sous leur forme propre et/ou sous la forme d'un ou plusieurs précurseurs.

Une première variante de l'invention consiste à introduire le promoteur halogéné décrit auparavant, tel quel, dans le mélange réactionnel.

Une seconde variante de mise en oeuvre, consiste à introduire ledit promoteur sous la forme d'au moins un précurseur.

Dans ce cas de figure, le précurseur se présente généralement sous la forme d'un composé susceptible de libérer dans le milieu réactionnel le radical du promoteur halogéné précité. Ceci a lieu par réaction du précurseur avec un halogène, l'hydracide correspondant, et/ou un iodure ; ces composés étant présents dans le milieu ou bien introduits à cet effet.

A titre d'exemple non limitatif de précurseurs convenables, on peut citer les composés choisis parmi les alcools de formule (1) ROH ; les éthers de formule (2) ROR' ou encore des esters de formules (3) R'COOR, utilisés seuls ou en mélange. Dans ces formules, les radicaux R et R', identiques ou différents, représentent chacun un radical alkyle en C₁-C₁₀, acyle en C₁-C₁₀, ou aryle en C₆-C₁₀ ; avec le radical R correspondant au radical du promoteur halogéné.

Ainsi, le méthanol, l'éthanol, le propanol, le butanol, le diméthyl éther, le diéthyl éther, l'oxyde d'éthylène, l'acétate de méthyle, sont notamment des précurseurs convenables du promoteur halogéné.

Habituellement la réaction de carbonylation est réalisée à une température comprise entre 150 et 250 °C. De préférence la température de réaction varie entre 180 et 210°C.

Selon un mode de réalisation particulier du procédé selon l'invention, le mélange réactionnel est purgé régulièrement des métaux de corrosion qu'il contient, dont notamment le fer, le molybdène, le chrome, le nickel. Cette opération est réalisée selon tout moyen connu de l'homme du métier, comme par exemple le traitement du mélange réactionnel par une résine échangeuse d'ions ou encore par précipitation du catalyseur et séparation de ce dernier, des métaux de corrosion, par filtration.

Le procédé selon l'invention convient à la fabrication de tout type d'acide carboxylique ou des esters correspondants, comprenant au minimum deux atomes de carbone. Ainsi, celui-ci peut être mis en oeuvre pour préparer l'acide propionique à partir de l'éthanol, l'acide succinique à partir de l'oxyde d'éthylène, l'acide adipique à partir du 1,4-butanediol, ou les esters correspondants à ces acides.

Cependant, ce procédé convient tout particulièrement à l'obtention d'acide acétique et/ou d'acétate de méthyle a partir de méthanol.

Selon un mode de réalisation préféré de l'invention, le procédé selon l'invention est mis en oeuvre à partir d'iodure de méthyle, d'acétate de méthyle, d'iodures sous forme soluble et d'acide acétique en tant que solvant, outre le méthanol.

Des exemples concrets mais non limitatifs de l'invention vont maintenant être présentés.

### EXEMPLES 1 ET 2

Ces essais de réaction de carbonylation ont été réalisés en continu dans un autoclave de 300 cm³ muni d'un moyen d'agitation mécanique et de moyens d'introduction des réactifs.

La solution catalytique utilisée est obtenue à partir de Ir₄CO₁₂ et est préparée comme suit :

On introduit dans un ballon en verre, 10 g de Ir₄CO₁₂, 50 g d'acide iodhydrique en solution à 57 % dans l'eau et 290 g d'acide acétique.

Le mélange est chauffé à reflux sous agitation et sous air pendant 4 heures.

Les introductions de méthanol, d'iodure de méthyle, d'acétate de méthyle et d'eau sont réglées de telle sorte que les teneurs des différents composants dans le milieu réactionnel soient maintenues comme indiquées dans le tableau ci-dessous.

Le temps de séjour dans le réacteur est d'environ 10 minutes.

La pression totale dans l'autoclave est de 30 bar et la température est maintenue à 190°C.

A la sortie de l'autoclave, le mélange réactionnel est dégazé et refroidi.

Le mélange et les gaz sont analysés par chromatographie en phase gazeuse.

| **Exemple** | **H**_{**2**}**O** | **CH**_{**3**}**CO**_{**2**}**CH**_{**3**} | **CH**_{**3**}**I** | **LiI** | **Ir** | **Li/Ir** | **V**_{**carb**} |
|---|---|---|---|---|---|---|---|
| 1 | 9,5 | 23 | 1,3 | 33 | 1200 | 0,7 | 8,5 |
| 2 | 5,8 | 13 | 4 | 61 | 1760 | 0,96 | 9,1 |

Les teneurs en eau, acétate de méthyle, iodure de méthyle sont exprimées en % poids, rapporté au poids total du mélange réactionnel, le complément à 100 % est apporté par l'acide acétique.

Les teneurs en iodure de lithium et en iridium sont exprimées en ppm.

V_{carb} représente la vitesse de carbonylation, exprimée en mol/l.h.

Elle est obtenue par mesure du débit de consommation du CO en tenant compte, en outre, de la quantité de ce gaz engagée dans la formation de CO₂.

### EXEMPLE 3

On traite 50 ml d'un mélange réactionnel issu d'une réaction de carbonylation, contenant majoritairement de l'acide acétique, un complexe soluble d'iridium (1800 ppm d'iridium), de l'iodure de méthyle, de l'acétate de méthyle, du méthanol, de l'eau et contenant 51 ppm de lithium sous forme d'iodure soluble dans une colonne de 2,7 cm de diamètre et d'une hauteur de 25 cm, dans laquelle on a disposé 26 ml de résine DOWEX C-500.

La résine a subi avant son utilisation des lavages successifs à l'eau et à l'isopropanol en vue d'éliminer les éventuels polluants organiques présents dans le produit commercial et a été immergée pendant 24 heures dans une solution d'acide acétique jusqu'à son gonflement maximal.

Le mélange réactionnel est alimenté à température ambiante sous pression atmosphérique en tête de colonne. La solution obtenue en pied de colonne est renvoyée en tête pour une nouvelle mise en contact avec la résine. Le traitement est poursuivi pendant 4 heures pour atteindre l'équilibre d'adsorption.

En fin de traitement, on récupère la solution pour l'analyser. Le dosage par absorption atomique indique qu'il reste 0,2 ppm de lithium sous forme d'iodure soluble dans la solution, ce qui correspond à un rendement d'adsorption de 97,3 %.
Par ailleurs, on constate que l'iridium n'est pas adsorbé sur la résine.

### EXEMPLE 4

On procède comme dans l'exemple précédent mais le mélange réactionnel est traité par la résine PUROLITE C 100.

L'analyse de la solution récupérée par adsorption atomique montre qu'il reste 0,45 ppm de lithium sous forme d'iodure soluble, ce qui correspond à un rendement d'adsorption de 94 %.

L'iridium n'est pas adsorbé sur la résine.

### EXEMPLE 5

On traite 50 ml d'un mélange réactionnel issu d'une réaction de carbonylation, contenant de l'acide acétique, un complexe soluble d'iridium (2100 ppm d'iridium), de l'iodure de méthyle, de l'acétate de méthyle, du méthanol, de l'eau et contenant 245 ppm de potassium sous forme d'iodure soluble.

On procède de la même façon que pour l'exemple 1.

L'analyse de la solution récupérée par adsorption atomique montre qu'il reste 0,3 ppm de potassium sous forme d'iodure soluble, ce qui correspond à un rendement d'adsorption supérieur à 99 %.

L'iridium n'est pas adsorbé sur la résine.

### EXEMPLE 6

On procède comme pour l'exemple précédent, excepté le fait que la résine utilisée est la résine PUROLITE C 100.

L'analyse montre qu'il reste 3,8 ppm de potassium sous forme d'iodure soluble, ce qui correspond à un rendement d'adsorption de 97 %. L'iridium n'est pas adsorbé.

### EXEMPLE 7

On effectue le traitement d'une solution catalytique d'iridium contenant 16500 ppm d'iridium, 76 ppm de sodium sous forme d'iodure soluble et 7 ppm de potassium sous forme d'iodure soluble.

Le traitement est réalisé par passage sur une résine AMBERLYST 16 (Rohm et Haas), selon le même mode opératoire que celui décrit dans les exemples précédents.

Le volume de résine employé est de l'ordre de 10 ml et le volume de solution catalytique traité est de l'ordre de 630 ml.

Les résultats montrent qu'il reste 5 ppm de sodium, correspondant à un rendement d'adsorption de 93 %, et 2 ppm de potassium, ce qui correspond à un rendement de 70%.

## Revendications

1. Procédé de préparation d'acides carboxyliques, ou des esters correspondants, présentant (n+1) atomes de carbone, par réaction en phase liquide, de monoxyde de carbone avec au moins un alcool présentant n atomes de carbone, en présence d'un système catalytique à base d'un composé de l'iridium et d'un promoteur halogéné, caractérisé en ce que l'on maintient dans le milieu, pendant la réaction, l'eau, dans une teneur variant entre 0 exclu et 10 %, le promoteur halogéné, dans une teneur variant entre 0 exclu et 10%, l'ester correspondant à l'acide carboxylique et à l'alcool précités, dans une teneur variant entre 2 et 40 %, des iodures sous forme soluble dans un quantité telle que le rapport atomique des iodures à l'iridium est compris entre 0 exclu et 10 ; l'acide carboxylique constituant le solvant de la réaction.

2. Procédé selon l'une quelconque des revendication précédentes, caractérisé en ce que l'on maintient le rapport atomique des iodures à l'iridium entre 0 exclu et 3 et de préférence entre 0 exclu et 1,5.

3. Procédé selon l'une quelconque des revendications précédentes, caractérisé en ce que l'on utilise des iodures de métal alcalin, de composés organo-phosphorés ou organo-azotés.

4. Procédé selon la revendication précédente, caractérisé en ce que l'on maintient dans le mélange une teneur en eau comprise entre 0,5 et 8 %.

5. Procédé selon l'une quelconque des revendications précédentes, caractérisé en ce que l'on maintient dans le mélange une teneur en promoteur halogéné comprise entre 0,5 et 8 %.

6. Procédé selon l'une quelconque des revendications précédentes, caractérisé en ce que l'on maintient dans le mélange une teneur en ester précité comprise entre 5 et 30 %.

7. Procédé selon l'une quelconque des revendications précédentes caractérisé en ce que l'on maintient le rapport atomique des iodures à l'iridium en effectuant les étapes suivantes :
- traitement par une résine échangeuse d'ions d'un mélange comprenant au moins le composé de l'iridium,
- addition d'iodures sous forme soluble dans une quantité telle que le rapport atomique des iodures à l'iridium dans le mélange réactionnel soit compris entre 0 exclu et 10.

8. Procédé selon la revendication 7, caractérisé en ce que l'on traite un mélange correspondant à la solution catalytique.

9. Procédé selon l'une quelconque des revendications 7 ou 8, caractérisé en ce que l'on traite un mélange correspondant au mélange réactionnel avant l'introduction des iodures solubles.

10. Procédé selon l'une quelconque des revendications 7 ou 9, caractérisé en ce que l'on traite le mélange réactionnel.

11. Procédé selon l'une quelconque des revendications 7 à 10, caractérisé en ce que l'on effectue le traitement avec une résine échangeuse de cations de type acide fort ou acide faible, sous la forme hydrogène.

12. Procédé selon la revendication précédente, caractérisé en ce que l'on effectue la traitement avec une résine de copolymères styrène-divinylbenzène présentant des groupements fonctionnels greffés sulfonés.

13. Procédé selon l'une des revendications précédentes, caractérisé en ce que l'on utilise un alcool hydrocarboné saturé en C₁-C₁₀.

14. Procédé selon l'une quelconque des revendications précédentes, caractérisé en ce que l'on utilise un promoteur halogéné comprenant un halogène choisi parmi le chlore le brome ou l'iode, en combinaison avec de l'hydrogène, un radical alkyle en C₁-C₁₀, un radical acyle en C₁-C₁₀ ou un radical aryle en C₆-C₁₀.

15. Procédé selon la revendication précédente, caractérisé en ce que l'on utilise un promoteur halogéné dont le radical correspond à celui de l'alcool précité.

16. Procédé selon l'une des revendications précédentes, caractérisé en ce que l'on prépare l'acide acétique par réaction du méthanol avec du monoxyde de carbone, en présence d'eau, d'iodure de méthyle, d'acétate de méthyle, d'iodures de métal alcalin, l'acide acétique constituant le solvant de la réaction.

## Patentansprüche

1. Verfahren zur Herstellung von Carbonsäuren oder entsprechenden Estern, die (n+1) Kohlenstoffatome aufweisen, durch Umsetzung in flüssiger Phase von Kohlenmonoxid mit mindestens einem Alkohol, der n Kohlenstoffatome aufweist, in Gegenwart eines katalytischen Systems auf Basis einer Iridiumverbindung und eines halogenierten Promotors, dadurch gekennzeichnet, daß man in dem Medium während der Reaktion einen Wassergehalt, der zwischen mehr als 0 und 10 % variiert, einen Gehalt an halogeniertem Promotor, der zwischen mehr als 0 und 10 % variiert, einen Gehalt an dem Ester, welcher der obengenannten Carbonsäure und dem obengenannten Alkohol entspricht, der zwischen 2 und 40 % variiert, und lodide in löslicher Form in einer solchen Menge aufrechterhält, daß das Atomverhältnis von lodiden zu Indium zwischen mehr als 0 und 10 liegt, wobei die Carbonsäure das Reaktionslösungsmittel darstellt.

2. Verfahren nach einem der vorhergehenden Ansprüche, dadurch gekennzeichnet, daß man das Atomverhältnis von lodiden zu Iridium zwischen mehr als 0 und 3 und vorzugsweise zwischen mehr als 0 und 1,5 hält.

3. Verfahren nach einem der vorhergehenden Ansprüche, dadurch gekennzeichnet, daß man Alkalimetalliodide und lodide von Organophosphorverbindungen oder Organostickstoffverbindungen verwendet.

4. Verfahren nach dem vorhergehenden Anspruch, dadurch gekennzeichnet, daß man in der Mischung einen Wassergehalt zwischen 0,5 und 8 % aufrechterhält.

5. Verfahren nach einem der vorhergehenden Ansprüche, dadurch gekennzeichnet, daß man in der Mischung einen Gehalt an halogeniertem Promotor zwischen 0,5 und 8 % aufrechterhält.

6. Verfahren nach einem der vorhergehenden Ansprüche, dadurch gekennzeichnet, daß man in der Mischung einen Gehalt an dem obengenannten Ester zwischen 5 und 30 % aufrechterhält.

7. Verfahren nach einem der vorhergehenden Ansprüche, dadurch gekennzeichnet, daß man das Atomverhältnis von lodiden zu Iridium in der Weise aufrechterhält, daß man die folgenden Stufen durchführt:
- Behandlung einer Mischung, die mindestens die Iridiumverbindung enthält, mit einem lonenaustauscherharz und
- Zugabe von lodiden in löslicher Form in einer solchen Menge, daß das Atomverhältnis von lodiden zu Iridium in der Reaktionsmischung zwischen mehr als 0 und 10 liegt.

8. Verfahren nach Anspruch 7, dadurch gekennzeichnet, daß man eine Mischung behandelt, die der katalytischen Lösung entspricht.

9. Verfahren nach einem der Ansprüche 7 oder 8, dadurch gekennzeichnet, daß man vor Einführung der löslichen lodide eine Mischung behandelt, die der Reaktionsmischung entspricht.

10. Verfahren nach einem der Ansprüche 7 oder 9, dadurch gekennzeichnet, daß man die Reaktionsmischung behandelt.

11. Verfahren nach einem der Ansprüche 7 bis 10, dadurch gekennzeichnet, daß man die Behandlung mit einem Kationenaustauscherharz vom Typ einer starken Säure oder einer schwachen Säure in der Wasserstoff-Form durchführt.

12. Verfahren nach dem vorhergehenden Anspruch, dadurch gekennzeichnet, daß man die Behandlung mit einem Harz aus Styrol/Divinylbenzol-Copolymeren durchführt, die aufgepfropfte funktionelle Sulfongruppen aufweist.

13. Verfahren nach einem der vorhergehenden Ansprüche, dadurch gekennzeichnet, daß man einen gesättigten C₁-C₁₀-Kohlenwasserstoffalkohol verwendet.

14. Verfahren nach einem der vorhergehenden Ansprüche, dadurch gekennzeichnet, daß man einen halogenierten Promotor verwendet, der ein Halogen, ausgewählt aus Chlor, Brom oder lod, in Kombination mit Wasserstoff, einem C₁-C₁₀-Alkylrest, einem C₁-C₁₀-Acylrest oder einem C₅-C₁₀-Arylrest enthält.

15. Verfahren nach dem vorhergehenden Anspruch, dadurch gekennzeichnet, daß man einen halogenierten Promotor verwendet, dessen Rest demjenigen des obengenannten Alkohols entspricht.

16. Verfahren nach einem der vorhergehenden Ansprüche, dadurch gekennzeichnet, daß man Essigsäure herstellt durch Umsetzung von Methanol mit Kohlenmonoxid in Gegenwart von Wasser, Methyliodid, Methylacetat und Alkalimetalliodiden, wobei die Essigsäure das Reaktionslösungsmittel darstellt.

## Claims

1. A process for preparing carboxylic acids or their corresponding esters containing (n+1) carbon atoms by reacting carbon monoxide with at least one alcohol containing n carbon atoms in the liquid phase, in the presence of a catalytic system based on an iridium compound and a halogenated promoter, characterized in that during the reaction, the water content is maintained at a level which is in the range 0 (exclusive limit) to 10%, the concentration of the halogenated promoter is maintained at a level in the range 0 (exclusive limit) to 10%, the concentration of the ester corresponding to the carboxylic acid and alcohol is maintained at a level which is in the range 2% to 40%, and the concentration of soluble iodides is maintained so that the atomic ratio of iodides to iridium is in the range 0 (exclusive limit) to 10, the carboxylic acid constituting the reaction solvent.

2. A process according to claim 1, characterized in that the atomic ratio of iodides to iridium is in the range 0 (exclusive limit) to 3, preferably in the range 0 (exclusive limit) to 1.5.

3. A process according to claim 1 or claim 2, characterized in that iodides of an alkali metal, organophosphorous compounds or organonitrogen compounds are used.

4. A process according to the preceding claim, characterized in that the water content in the mixture is maintained in the range 0.5% to 8%.

5. A process according to any one of the preceding claims, characterized in that the concentration of halogenated promoter in the mixture is maintained in the range 0.5% to 8%.

6. , A process according to any one of the preceding claims, characterized in that the concentration of said ester in the mixture is maintained in the range 5% to 30%.

7. A process according to any one of the preceding claims, characterized in that the atomic ratio of iodides to iridium is maintained by carrying out the following steps:
• treatment of a mixture containing at least the iridium compound with an ion exchange resin;
• addition of soluble iodides in a quantity such that the atomic ratio of iodides to iridium in the reaction mixture is in the range 0 (exclusive limit) to 10.

8. A process according to claim 7, characterized in that a mixture which corresponds to the catalytic solution is treated.

9. A process according to claim 7 or claim 8, characterized in that a mixture which corresponds to the reaction mixture before introduction of the soluble iodides is treated.

10. A process according to any one of claims 7 to 9, characterized in that the reaction mixture is treated.

11. A process according to any one of claims 7 to 10, characterized in that treatment is carried out using a strong acid type or a weak acid type cationic exchange resin, in its hydrogen form.

12. A process according to the preceding claim, characterized in that treatment is carried out with a styrene-divinylbenzene copolymer resin containing grafted sulphonated functional groups.

13. A process according to any one of the preceding claims, characterized in that a saturated C₁-C₁₀ hydrocarbon alcohol is used.

14. A process according to any one of the preceding claims, characterized in that a halogenated promoter is used which contains a halogen selected from chlorine, bromine or iodine, in combination with hydrogen, a C₁-C₁₀ alkyl radical, a C₁-C₁₀ acyl radical or a C₆-C₁₀ aryl radical.

15. A process according to the preceding claim, characterized in that a halogenated promoter is used in which the radical corresponds to that of said alcohol.

16. A process according to any one of the preceding claims, characterized in that acetic acid is prepared by reacting methanol with carbon monoxide in the presence of water, methyl iodide, methyl acetate and alkali metal iodides, the acetic acid constituting the reaction solvent.
